Europäisches Patentamt

European Patent Office

Office européen des brevets

(19)

(11) Veröffentlichungsnummer: **0 111 783**

**A1**

(12) # EUROPÄISCHE PATENTANMELDUNG

(21) Anmeldenummer: 83111970.6

(22) Anmeldetag: 29.11.83

(51) Int. Cl.³: **C 12 M 1/18**
**C 12 M 1/28**

(30) Priorität: 10.12.82 CH 7217/82

(43) Veröffentlichungstag der Anmeldung:
27.06.84 Patentblatt 84/26

(84) Benannte Vertragsstaaten:
AT BE CH DE FR GB IT LI NL SE

(71) Anmelder: F. HOFFMANN-LA ROCHE & CO.
Aktiengesellschaft

CH-4002 Basel(CH)

(72) Erfinder: Schmidt, Bernd
Burgackerweg 13
D-7889 Grenzach-Wyhlen(DE)

(74) Vertreter: Lederer, Franz, Dr. et al,
Patentanwälte Dr. Lederer Franz Meyer-Roxlau Reiner F.
Lucile-Grahn-Strasse 22
D-8000 München 80(DE)

(54) **Mit Nährmedium zu füllendes oder gefülltes Behältnis.**

(57) Es wird ein transparentes Behältnis beschrieben, welches mehrere Kammern aufweist, die mit einem festen Nährmedium gefüllt sind oder zu füllen sind. Charakteristisch für dieses Behältnis ist, dass die Oeffnung einer Kammer nach oben zeigt, während die Oeffnung einer benachbarten Kammer nach unten zeigt. Durch diese Massnahme wird eine Diffusionsbarriere erreicht. Als zusätzliche Diffusionsbarriere können die Aussenseiten der Kammern aufgerauht sein.

EP 0 111 783 A1

RAN 4090/142

F.HOFFMANN-LA ROCHE & CO. Aktiengesellschaft, Basel (Schweiz)

## Mit Nährmedium zu füllendes oder gefülltes Behältnis

Die vorliegende Erfindung betrifft ein in der mikrobiologischen Diagnostik zu verwendendes, transparentes Behältnis, welches mit festem Nährmedium zu füllen bzw. gefüllt ist.

In der mikrobiologischen Diagnostik ist es häufig notwendig, in speziellen festen Nährmedien, z.B. in Agaren, entstehende Aufhellungszonen oder Farbveränderungen zum Nachweis von Mikroorganismen speziell zu beobachten (z.B. Hämolysezonen, Aufhellungszonen lipolytischer Reaktionen). Für eine sichere Auswertung müssen aus naheliegenden Gründen transparente Gefässe verwendet werden. Werden mehrere solcher Gefässe nebeneinander angeordnet, so besteht die Gefahr, dass Nährbodenbestandteile vom einen Gefäss über die Wand in das benachbarte Gefäss diffundieren können.

Es war Aufgabe der vorliegenden Erfindung, ein Behältnis mit mehreren Kammern enthaltend festes Nährmedium zu schaffen, das den obenerwähnten Nachteil nicht aufweist.

Klt/5.9.83

Erfindungsgemäss wurde diese Aufgabe dadurch gelöst, dass die einzelnen Kammern so angeordnet sind, dass die Oeffnung einer Kammer nach oben zeigt, während diejenige einer benachbarten Kammer nach unten zeigt. Die Kammern können hierbei beliebig oft nebeneinander angeordnet werden.

Die vorliegende Erfindung betrifft demnach ein mit festem Nährmedium zu füllendes oder gefülltes, transparentes Behältnis, welches dadurch gekennzeichnet ist, dass es mehrere Kammern aufweist, wobei die Oeffnung einer Kammer nach oben zeigt, während die Oeffnung einer benachbarten Kammer nach unten zeigt.

Als festes Nährmedium für das erfindungsgemässe Behältnis kommt insbesondere Agar in Betracht, wobei gleiche oder unterschiedliche Agare verwendet werden können.

Das transparente Behältnis besteht vorzugsweise aus einem Plastikmaterial, wie Polystyrol oder Polyäthylen.

Obwohl die Diffusion von einer Kammer in die andere praktisch ausgeschlossen ist, kann man als weitere Diffusionsbarriere die Aussenseiten der Kammern aufrauhen.

Ein besonders bevorzugtes Behältnis gemäss vorliegender Erfindung besteht aus zwei Kammern und ist mit dem Verschluss eines Röhrchens verbunden, in das es hineinragt.

Besonders bevorzugt ist deshalb im Rahmen der vorliegenden Erfindung eine Vorrichtung zum Nachweisen von Mikroorganismen, bestehend aus einem mit einem Verschluss versehenen durchsichtigen Röhrchen, an dessen Verschluss ein Träger fixiert ist, welcher in das Röhrchen hineinragt, und welcher zwei Kammern aufweist, welche dadurch gekennzeichnet ist, dass die eine Kammeröffnung nach oben zeigt, während die andere Kammeröffnung nach unten zeigt.

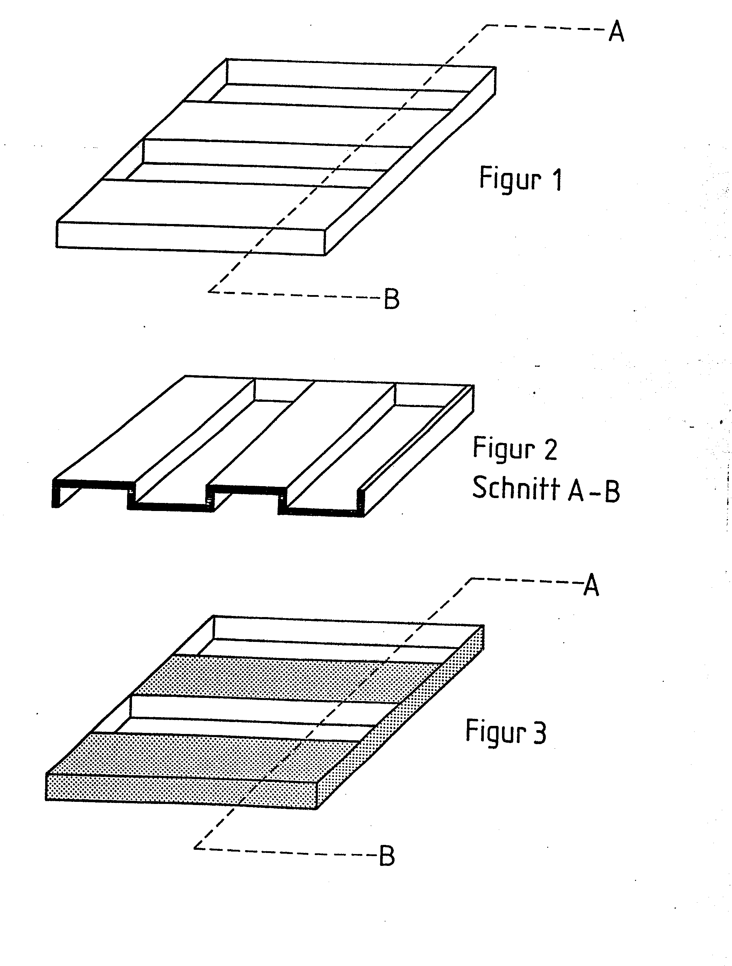

Figur 1 zeigt eine perspektivische Darstellung eines erfindungsgemässen Behältnisses aus Polystyrol mit 4 Kammern.

Figur 2 zeigt den Schnitt entlang der Linie A - B.

Figur 3 zeigt eine perspektivische Darstellung eines erfindungsgemässen Behältnisses aus Polystyrol mit 4 Kammern, deren Aussenseiten aufgerauht sind, was durch die punktierten Flächen verdeutlicht wird.

Ein solches Behältnis mit vier oder x-beliebigen Kammern eignet sich insbesondere für das Laboratorium. Beim Füllen der Kammern werden zuerst diejenigen gefüllt, deren Oeffnungen nach oben zeigen, wonach nach Erstarren des Agars das Behältnis umgedreht wird, worauf die andern Kammern gefüllt werden.

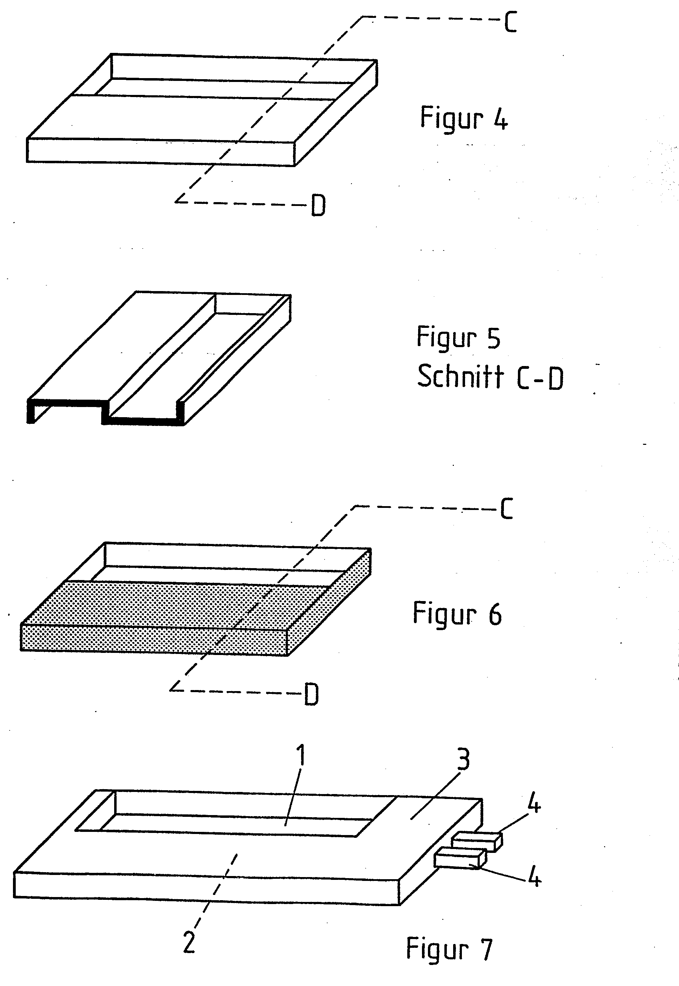

Figur 4 zeigt eine perspektivische Darstellung eines erfindungsgemässen Behältnisses aus Polyäthylen mit 2 Kammern.

Figur 5 zeigt den Schnitt entlang der Linie C - D.

Figur 6 zeigt eine perspektivische Darstellung eines erfindungsgemässen Behältnisses aus Polyäthylen mit 2 Kammern, deren Aussenseiten aufgerauht sind, was durch die punktierten Flächen verdeutlicht wird.

Figur 7 zeigt das Behältnis gemäss Figur 4 mit Mitteln zur Fixierung an den Schraubverschluss eines Röhrchens. Vor den beiden Kammern (1,2) ist eine plane Polyäthylenplatte (3) ausgebildet, die mit zwei Stiften (4) versehen ist, welche in zwei entsprechende Vertiefungen des Schraubverschlusses passen.

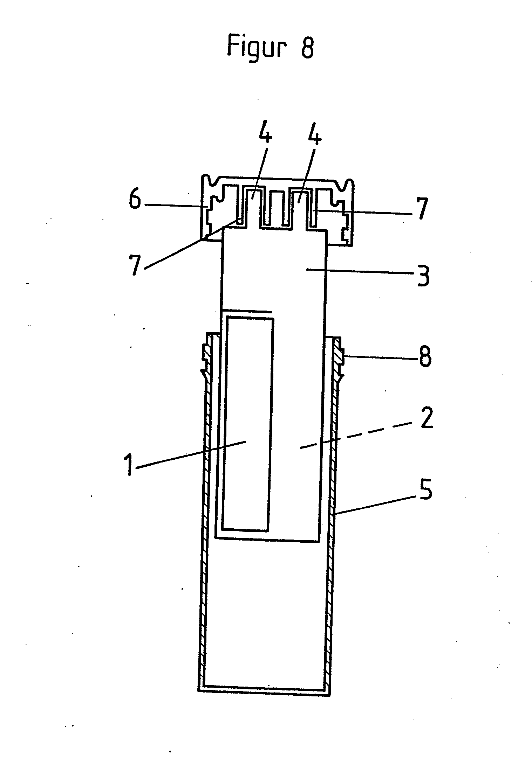

Figur 8 zeigt das Röhrchen (5) mit Schraubverschluss (6) mit den Vertiefungen (7) und den sich darin befindlichen Stiften (4), der planen Polyäthylenplatte (3) und den beiden Nährbodenkammern (1) und (2). Beim Schliessen des Röhrchens wird der Schraubverschluss (6) auf das Schraubgewinde (8) aufgeschraubt.

Das Behältnis nach Figur 8 eignet sich insbesondere für den Transport und die anschliessende Untersuchung der Mikroorganismen im Laboratorium.

Die Füllung der beiden Kammern erfolgt gleich wie das Füllen eines Behältnisses mit 4 oder mehr Kammern. Vorzugsweise erfolgt die Füllung vollautomatisch.

Patentansprüche

1. Mit festem Nährmedium zu füllendes oder gefülltes, transparentes Behältnis, dadurch gekennzeichnet, dass es mehrere Kammern aufweist, wobei die Oeffnung einer Kammer nach oben zeigt, während die Oeffnung einer benachbarten Kammer nach unten zeigt.

2. Behältnis nach Anspruch 1, dadurch gekennzeichnet, dass das Nährmedium Agar ist.

3. Behältnis nach Anspruch 1 oder 2, dadurch gekennzeichnet, dass das Behältnis aus Plastikmaterial besteht.

4. Behältnis nach Anspruch 3, dadurch gekennzeichnet, dass das Plastikmaterial Polystyrol ist.

5. Behältnis nach Anspruch 3, dadurch gekennzeichnet, dass das Plastikmaterial Polyäthylen ist.

6. Behältnis nach einem der Ansprüche 1 bis 5, dadurch gekennzeichnet, dass die Aussenseiten der Kammern aufgerauht sind.

7. Behältnis nach einem der Ansprüche 1 bis 6, dadurch gekennzeichnet, dass es aus zwei Kammern besteht.

- 6 -                    **0111783**

8. Behältnis nach Anspruch 7, dadurch gekennzeichnet, dass es mit dem Verschluss eines durchsichtigen Röhrchens verbunden ist.

9. Behältnis nach Anspruch 8, dadurch gekennzeichnet, dass es sich im Innern eines durchsichtigen Röhrchens befindet.

10. Vorrichtung zum Nachweisen von Mikroorganismen, bestehend aus einem mit einem Verschluss versehenen durchsichtigen Röhrchen, an dessen Verschluss ein Träger fixiert ist, welcher in das Röhrchen hineinragt, und welcher zwei Kammern aufweist, die mit demselben oder verschiedenen festen Nährböden gefüllt sind, dadurch gekennzeichnet, dass die eine Kammeröffnung nach oben zeigt, während die andere Kammeröffnung nach unten zeigt.

\*\*\*

Figur 1

Figur 2
Schnitt A-B

Figur 3

F.Hoffmann-La Roche & Co.AG
RAN 4090/142

0111783

Figur 4

Figur 5
Schnitt C-D

Figur 6

Figur 7

F.Hoffmann-La Roche & Co.AG

Figur 8

F.Hoffmann-La Roche & Co.AG

## EUROPÄISCHER RECHERCHENBERICHT

**Europäisches Patentamt**

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | US-A-3 961 696 (B.A. BOWIE et al.) <br> * Spalte 2, Zeilen 3-15; Figuren 1,2 * | 1-3,6-10 | C 12 M 1/18 <br> C 12 M 1/28 |
| Y | | 4,5 | |
| | --- | | |
| X | DE-A-2 936 294 (A. RANTAMA) <br><br> * Seite 3, Zeilen 1-8; Seite 4, Zeilen 30-39; Seite 5, Zeilen 1-33; Patentansprüche 1-5; Figuren 1,2 * | 1-3,7-10 | |
| | --- | | |
| Y | FR-A-2 264 089 (ORION) <br> * Seiten 7,8 * | 4,5 | |
| | --- | | RECHERCHIERTE SACHGEBIETE (Int. Cl. 3) |
| A | GB-A-1 499 265 (J.M. SCHMIDT) | | C 12 M <br> B 01 L |
| | --- | | |
| A | FR-A-2 381 102 (H. FORRER et al.) | | |
| | ----- | | |

Der vorliegende Recherchenbericht wurde für alle Patentansprüche erstellt.

| Recherchenort | Abschlußdatum der Recherche | Prüfer |
|---|---|---|
| DEN HAAG | 29-02-1984 | COUCKE A.O.M. |